# EUROPEAN PATENT SPECIFICATION

(11) **EP 3 660 002 B1**
(45) Date of publication and mention of the grant of the patent: **17.02.2021**
(21) Application number: 19211707.5
(22) Date of filing: 27.11.2019
(51) Int. Cl.: C07D 223/10, C07D 201/04, B01J 19/24, B01J 8/02

(54) **METHOD FOR ISOLATING CAPROLACTAM FROM THE UPPER LACTAM PHASE COMPRISING THE STEPS OF 1) DISTILLATION, 2) REFINING WITH HYDROGEN IN THE PRESENCE OF A NOBEL METAL CATALYST AND 3) RECTIFICATION**
VERFAHREN ZUR ISOLIERUNG VON CAPROLACTAM AUS DER OBEREN LACTAM-PHASE UMFASSEND DIE SCHRITTE DER 1) DESTILLATION, 2) REINIGUNG MIT WASSERSTOFF IN GEGENWART EINES EDELMETALLKATALYSATORS UND 3) REKTIFIKATION
PROCÉDÉ POUR ISOLER CAPROLACTAME DE LA PHASE DE LACTAME SUPÉRIEURE COMPRENANT LES ÉTAPES DE 1) DISTILLATION, 2) RAFFINAGE AVEC D'HYDROGÈNE EN PRÉSENCE D'UN CATALYSEUR À BASE DE MÉTAL NOBLE ET 3) RECTIFICATION

(30) Priority: 27.11.2018 CZ 20180651
(43) Date of publication of application: 03.06.2020
(73) Proprietor: Vysoká skola chemicko-technologická v Praze, 160 00 Praha 6, Dejvice (CZ)
(72) Inventor: Trejbal, Jiri, 278 01 Kralupy nad Vltavou (CZ); Zapletal, Martin, 755 01 Vsetin (CZ)
(74) Representative: Musil, Dobroslav

(56) References cited:
- US-A1- 2016 237 038

## Description

### Technical field

The invention relates to a method for the isolation of ε-caprolactam from the upper lactam phase obtained by separation from the sulphate phase after neutralization of the reaction mixture obtained from the Beckmann cyclohexyl oxime rearrangement with ammonia.

### Background art

The starting materials for the synthesis of ε-caprolactam are cyclohexanone and hydroxylamine, which react together to form oxime. This oxime is then contacted with 30% oleum (a solution of SO₃ in H₂SO₄), and in this very acidic environment, the so-called Beckmann rearrangement occurs to form ε-caprolactam. After the rearrangement, the lactam sulfuric acid is neutralized with an ammonia solution and the resulting mixture is separated at the bottom of a separate liquid phase. Depending on the rearrangement and subsequent neutralization conditions, the upper lactam phase contains 20 to 40 % by weight of water, 0.05 to 2 % by weight of ammonium sulfate and 0.1 to 2 % by weight of non-volatile organic substances. The lower sulfate phase contains 30 to 35 % by weight of ammonium sulfate and up to 1 % by weight of ε-caprolactam.

This synthesis of ε-caprolactam has a high yield - over 99 %, but produces a number of minor substances that need to be removed to guarantee the desired quality. In the case of ε-caprolactam, the quality indicator being monitored is not a content which is always higher than 99.99 %, but other parameters. First, it is the absorbance of a 50% aqueous solution of ε-caprolactam for light with a wavelength of 290 nm, the absorbance of which must be less than 0.05 when using a 10 mm cuvette. Also, the permanganate index of a 3% aqueous solution of ε-caprolactam is monitored, where a maximum value of 4.4 is required. In addition, the volatile base content, which must be less than 0.4 meq/kg, is monitored, as well as the colour according to Hazen (APHA) of the 50% aqueous solution of ε-caprolactam, which must be less than 5.

The basic method for the isolation of ε-caprolactam from the upper lactam phase has stabilized in the fifties of the 20th century and is still applied in various modifications. The principle of removing undesirable substances is double extraction by means of a suitable solvent and water. First, ε-caprolactam is extracted from the upper lactam phase into an organic solvent and then the organic solution thus formed is extracted with water into which ε-caprolactam is transferred. Thereafter, the aqueous solution is freed from water by distillation and ε-caprolactam is distilled as a product under high vacuum, usually on rotor evaporators.

Trichlorethylene (DD 9215) and benzene (DE 1194863) are practically exclusively used as organic solvents for extraction. However, with respect to the toxicity of both substances, there is general pressure to replace them. Other suitable solvents which are taken into consideration are, for example, toluene (PL 210210) or mixtures made up of more substances (PL 381645). The disadvantage of these new systems, on the other hand, is lower extraction capacity than that of trichlorethylene and benzene and their more demanding regeneration.

To improve the quality of ε-caprolactam, it is recommended that the extraction step should be followed by hydro-refining of the aqueous solution usually on nickel catalysts (DE 3925575).

U.S. Pat. No. 2016237038A1 discloses multi-step process consists from Beckman rearrangement of cyclohexyl oxime in the presence of sulfuric acid, neutralization of the reaction mixture by alkali to obtain a caprolactam rich water solution, extraction of the caprolactam water solution by organic solvent as benzene, toluene or dichloroethane, hydrogenation of caprolactam - organic solvent solution on the noble metal catalyst and finally recovery of organic solvent and caprolactam isolation by vacuum distillation.

However, it is becoming more and more obvious that the extractive process, even when using a very efficient rectification column and high reflux ratios and even in combination with hydro-refining, is not capable of removing minor substances from the final product. Although the content of the minor substances is in the range of units of ppm, which makes it difficult to determine them analytically at all, they have a significant negative effect on the ε-caprolactam quality, especially in terms of absorbance and volatile base content.

The object of the invention is to propose a method for the isolation of ε-caprolactam from the upper lactam phase, which would result in the isolation of a pure product which would meet the required quality criteria, this being achieved without using an extraction step.

### Principle of the invention

The object of the invention is achieved by a method for the isolation of ε-caprolactam without using extraction from the upper lactam phase obtained by separation from the sulphate phase after neutralization of the reaction mixture obtained from the Beckmann cyclohexyl oxime rearrangement with ammonia. This upper lactam phase, which contains 20 to 40 % by weight of water, 0.05 to 2 % by weight of ammonium sulfate and 0.1 to 2 % by weight of non-volatile organic substances is first distilled by simple distillation under reduced or atmospheric pressure, thereby reducing the water content to 5 to10 % by weight. Thereafter, the solid phase is mechanically removed from it (e.g. by sedimentation, filtration, centrifugation, etc.), the solid phase being produced by distillation and being largely composed of ammonium sulphate, which is generally known to act as a catalyst for (undesired) polymerization of ε-caprolactam.

The concentrated mixture thus obtained is subsequently distilled at a pressure of 0.1 to 4 kPa, whereby non-volatile components are removed from it. In a preferred variant, the concentrated mixture is distilled at a pressure of 0.3 to 1 kPa, since for a pressure of less than 0.3 kPa, the cost of its formation increases without further benefit and, at a pressure greater than 1 kPa, undesirable decomposition and polymerization may occur.

In both distillations, it is advantageous in terms of efficiency if the distillate is condensed partially.

Thereafter, in the distillate thus prepared by the addition of at least one solvent selected from the group consisting of water, methanol, ethanol, propanol, isopropyl alcohol, or of any mixture of at least two of them, the ε-caprolactam concentration is adjusted to 30 to 70 % by weight and the mixture thus obtained is refined by hydrogen at a temperature of 100 to 150 °C and a hydrogen pressure of 1 to 10 MPa in the presence of a catalyst based on at least one noble metal selected from the group consisting of Pd, Pt, Ru. ε-caprolactam concentrations higher than 70 % by weight result in a dramatic reduction in the catalyst activity, on the other hand, concentrations below 30 % by weight have a negative impact on the economy of the process. A preferred compromise with respect to the catalyst life and recycling costs is ε-caprolactam concentration ranging from 40 to 60 % by weight. The hydro-refining process reduces the multiple bonds of the impurities present, which absorb radiation at a wavelength of 290 nm, which has a positive effect on the ε-caprolactam quality achieved. A suitable catalyst can be, in particular, the so-called supported heterogeneous catalyst which contains a support, for example based on Al₂O₃ or SiO₂, on whose surface at least one of the above-mentioned noble metals is deposited. The total amount of the noble metal/metals is 0.1 to 5 % of weight of this catalyst, preferably 1 to 3 % of weight of the catalyst. If the amount of noble metal/metals is/are below 1 % of weight of the catalyst, the activity of the catalyst is relatively low and a great amount of the catalyst is required. If the amount of noble metal/metals content exceeds 3 % of weight of the catalyst, the catalyst activity increases only negligibly; however, this does not apply to its price. The hydro-refining process is preferably, but not necessarily, carried out in a trickle bed reactor. In a preferred embodiment, the supported heterogeneous catalyst is in the form of extrudates, pellets or tablets with the surface coating of the active ingredient (the so-called "egg shell"), etc. The particle size of the support is preferably from 1 to 5 mm. Smaller particles have high hydraulic resistance and are very difficult to access, while larger particles have an unfavourable surface/volume ratio. The nickel catalyst used in extraction methods has proved totally unsuitable in this process.

The raffinate thus prepared is subsequently rectified in a series of at least two high-efficiency rectification columns - for example, columns equipped with structured packing with the lowest liquid residence time. During the rectification, light fractions and heavy residues are separated from this raffinate, thereby increasing ε-caprolactam concentration and improving its qualitative parameters. The rectification is performed at a pressure of 0.05 to 4 kPa, preferably 0.1 to 2 kPa, when for a pressure of less than 0.1 kPa, the cost of its formation increases and at a pressure greater than 2 kPa, undesirable decomposition and polymerization may occur. The minimum required efficiency of the rectification column system is 10 theoretical plates.

The light fractions and heavy residues separated by the rectification are preferably recycled to the upper lactam phase, thereby increasing the efficiency of the subsequent isolation of ε-caprolactam.The proportion of the light fractions and heavy residues thus recycled is with respect to the economic viability of this step at least 5 %, preferably from 50 to 100 %.

As can be seen from the following examples, the method for the isolation of ε-caprolactam from the upper lactam phase according to the present invention more than fully replaces the conventionally used method based on the extraction of ε-caprolactam with an organic solvent and subsequent extraction into water, as by this method ε-caprolactam of a considerably higher quality is obtained, this with lower investment and operating costs and without the use of volatile, toxic or flammable solvents.

### Examples of embodiment

### Example 1

50 kg of the upper lactam phase with an initial water content of 30.5 % by weight, ammonium sulfate 0.95 % by weight and non-volatile organic substances 0.5 % by weight was dewatered to a water content of 10 % by weight by simple distillation at atmospheric pressure. Thereafter, the solid phase which was formed during the distillation was removed by filtration and the concentrated mixture thus obtained was distilled at a pressure of 0.1 kPa by partial condensation. The 0.4 kg of distillate thus formed, which contained 98.4 % of ε-caprolactam, was then diluted with distilled water to a ε-caprolactam concentration of 70 % by weight. The mixture thus formed was subsequently refined by hydrogen at a temperature of 100 ° C and a hydrogen pressure of 10 MPa and a 15 l/h feed rate in a 50 mm internal diameter reactor with a 2 m long scrubbed catalyst layer, the catalyst consisting of Al₂O₃ tablets having a size of 5x5 mm and a palladium content of 3 %. The raffinate thus obtained was subsequently batch rectified in a series of two rectification columns, each of them having an efficiency of 10 theoretical plates, at a pressure of 0.05 kPa.

The qualitative parameters of the product thus obtained are given in table 1.

### Example 2

The upper lactam phase was processed in the same manner as in Example 1, except that the distillate was diluted to a ε-caprolactam concentration of 50 % by weight and then was rectified without previous hydro-refining.

The qualitative parameters of the product thus obtained are given in table 1.

### Example 3

The upper lactam phase was processed in the same manner as in Example 1, except that the raffinate obtained by hydro-refining was batch rectified in a rectification column with an efficiency of 5 theoretical plates at a pressure of 1 kPa.

The qualitative parameters of the product thus obtained are given in table 1.

### Example 4

The upper lactam phase was processed in the same manner as in Example 1, except that the concentrated mixture was distilled at a pressure of 4 kPa after removal of the solid phase. The raffinate obtained by hydro-refining was then rectified in a series of two rectification columns, each of them having an efficiency of 5 theoretical plates, at a pressure of 4 kPa.

The qualitative parameters of the product thus obtained are given in table 1.

### Example 5

The upper lactam phase was processed in the same manner as in Example 1 except that it was distilled at a pressure of 10 kPa to reduce the water content to 5 % by weight.

The qualitative parameters of the product thus obtained are given in table 1.

### Example 6

The upper lactam phase was treated in the same manner as in Example 1, except that the hydro-refining process took place at a temperature of 120 °C, a hydrogen pressure of 1 MPa and a feed rate of 10 l/h in a 50 mm internal diameter reactor with a 2 m long scrubbed catalyst layer, the catalyst consisting of SiO₂ extrudates having a size of 1x1 mm and a platinum content of 5 %.

The qualitative parameters of the product thus obtained are given in table 1.

### Example 7

The upper lactam phase was processed in the same manner as in Example 1, except that the concentrated mixture was diluted with distilled water and methanol to a ε-caprolactam concentration of 30 % by weight after removal of the non-volatile substances, with a methanol content of 20 % by weight in the mixture obtained. The mixture thus prepared was subsequently refined by hydrogen at a temperature of 150 °C, a hydrogen pressure of 5 MPa and a 20 l/h feed rate in a 50 mm internal diameter reactor with a 2 m long scrubbed catalyst layer, the catalyst consisting of Al₂O₃ tablets having a size of 3x3 mm and a palladium content of 1 %. The raffinate obtained by the hydro-refining process was subsequently rectified in a series of two rectification columns, each of them having an efficiency of 10 theoretical plates, at a pressure of 2 kPa.

The qualitative parameters of the product thus obtained are given in table 1.

### Example 8 - extraction procedure

The upper lactam phase was countercurrently extracted with trichlorethylene to a 20% solution of ε-caprolactam in trichlorethylene. This solution was then countercurrently extracted with water to a 38% solution of ε-caprolactam in water, which was then dewatered at atmospheric pressure to a water content of 7 % by weight. The solution thus obtained was batch rectified in a rectification column with an efficiency of 10 theoretical plates, at a pressure of 1 kPa.

The qualitative parameters of the product thus obtained are given in table 1.

### Example 9 - extraction procedure supplemented by hydro-refining

The upper lactam phase was countercurrently extracted with trichlorethylene to a 20% solution of ε-caprolactam in trichlorethylene. This solution was then countercurrently extracted with water to a 38% solution of ε-caprolactam in water, which was then dewatered at atmospheric pressure to a water content of 6 % by weight. The solution thus obtained was refined by hydrogen at a temperature of 130 °C, a hydrogen pressure of 5 MPa and a feed rate of 15 l/h in a 50 mm internal diameter reactor with a 2 m long scrubbed catalyst layer, the catalyst consisting of a Al₂O₃ tablets having a size of 3x3 mm a palladium content of 5 %. The obtained raffinate was batch rectified at a pressure of 1 kPa in a series of two rectification columns, each column having an efficiency of 10 theoretical plates.

The qualitative parameters of the product thus obtained are given in table 1.

**Table 1**

| Example | Absorbance (max. 0.05) | P-index (max. 4.4) | Volatile bases (max. 0.4 meq/kg) | Colour (max. 5 HU) |
|---|---|---|---|---|
| 1 | 0.015 | 1.3 | 0.15 | 3 |
| 2 | 1.215 | 48.3 | 0.83 | 7 |
| 3 | 0.016 | 1.5 | 0.45 | 4 |
| 4 | 0.035 | 2.6 | 0.16 | 5 |
| 5 | 0.014 | 1.3 | 0.14 | 3 |
| 6 | 0.018 | 1.5 | 0.25 | 4 |
| 7 | 0.020 | 1.4 | 0.16 | 4 |
| 8 | 0.045 | 4.3 | 0.35 | 5 |
| 9 | 0.035 | 2.5 | 0.28 | 5 |

It is apparent from the above-mentioned examples that the method for the isolation of ε-caprolactam from the upper lactam phase according to the present invention produces ε-caprolactam of a significantly higher quality than the conventionally used process based on the extraction of ε-caprolactam with an organic solvent and subsequent extraction into water, as well as the process of DE 3925575, in which the hydro-refining is performed after extraction and before final rectification.

## Claims

1. A method for the isolation of ε-caprolactam from the upper lactam phase obtained by separation from the sulphate phase after neutralization of the reaction mixture obtained from the Beckmann cyclohexyl oxime rearrangement with ammonia, **characterized in that** the upper lactam phase is first distilled at a reduced or atmospheric pressure, by which means the water content in it is reduced to 5 to 10 % by weight, whereupon the solid phase formed by distillation is mechanically removed therefrom and the concentrated mixture thus obtained is distilled at a pressure of 0.1 to 4 kPa, whereby non-volatile components are removed therefrom, then in the distillate thus obtained the ε-caprolactam concentration is adjusted to 30 to 70 % by weight by adding at least one solvent selected from the group consisting of water, methanol, ethanol, propanol, isopropyl alcohol, or a mixture of at least two of them, and the mixture thus formed is refined by hydrogen at a temperature of 100 to 150 °C and a hydrogen pressure of 1 to 10 MPa in the presence of a supported heterogeneous catalyst which comprises at least one noble metal selected from the group consisting of Pd, Pt, Ru, whereby the multiple bonds of the impurities present, which absorb radiation at a wavelength of 290 nm, are reduced, and afterwards, the raffinate thus obtained is rectified in a series of at least two rectification columns operating at a pressure of 0.05 to 4 kPa with a cumulative minimum column system efficiency of 10 theoretical plates, whereby light fractions and heavy residues are separated from the raffinate by rectification, thereby isolating ε-caprolactam therefrom.

2. The method according to claim 1, **characterized in that** the concentrated mixture is distilled at a pressure of 0.3 to 1 kPa after removal of the solid phase.

3. The method according to claim 1, **characterized in that** the concentration of ε-caprolactam in the distillate is adjusted by the addition of solvent to 40 to 60 % by weight.

4. The method according to claim 1, **characterized in that** the raffinate is rectified in a series of rectification columns operating at a pressure of 0.1 to 2 kPa.

5. The method according to claim 1, **characterized in that** at least 5 % of the light fractions and heavy residues separated from the raffinate by rectification are recycled to the upper lactam phase for further isolation of ε-caprolactam.

6. The method according to claim 1, **characterized in that** the distillate is condensed partially during the distillation.

7. The method according to claim 1, **characterized in that** the hydro-refining process is performed in the presence of a supported heterogeneous catalyst comprising a support based on Al₂O₃ or SiO₂, on which at least one noble metal selected from the group consisting of Pd, Pt, Ru is deposited, the total amount of the noble metal/metals being from 0.1 to 5 % of the catalyst weight.

8. The method according to claim 7, **characterized in that** the total amount of the noble metal/metals represents from 1 to 3 % by weight of the catalyst.

9. The method according to claim 1, **characterized in that** the hydro-refining process takes place in a trickle bed reactor.

10. The method according to claim 7 or 8, **characterized in that** the catalyst support consists of particles having a size of 1 to 5 mm.

## Patentansprüche

1. Verfahren zur Isolation eines ε-Caprolaktams von einer oberen Laktamphase, die durch Trennung von einer Sulfatphase nach einer Neutralisierung einer Reaktionsmischung gewonnen wird, die aus der Beckmann-Umlagerung von Zyklohexyloxim durch Ammoniak gewonnen wird, **dadurch gekennzeichnet, dass** diese obere Laktamphase zuerst unter einem erniedrigten oder atmosphärischen Druck destilliert wird, wodurch der Wasserinhalt darin auf 5 bis 10 % Gew. reduziert wird, danach daraus die feste Phase mechanisch beseitigt wird, die bei einer Destillation entsteht, und die so gewonnene konzentrierte Mischung beim Druck von 0,1 bis 4 kPa destilliert wird, wobei daraus die nicht flüchtigen Bestandteile beseitigt werden, in dem so gewonnenen Destillat durch einen Zusatz von mindestens einem Lösungsmittel der Gruppe Wasser, Methanol, Ethanol, Propanol, Iso-Propylalkohol, oder Mischung von mindestens zwei von ihnen, die Konzentration von ε-Caprolaktam auf 30 bis 70 % Gew. aufbereitet wird und die so gebildete Mischung bei der Temperatur von 100 bis 150 °C und Wasserstoffdruck von 1 bis 10 MPa in der Anwesenheit eines heterogenen Trägerkatalysators hydrierraffiniert wird, der mindestens ein Edelmetall der Gruppe Pd, Pt, Ru enthält, wobei es zur Reduktion von Mehrfachbindungen von anwesenden Verunreinigungen kommt, die eine Strahlung bei einer Wellenlänge von 290 nm absorbieren, und das so gebildete Raffinat dann in Serie von mindestens zwei Rektifikationskolonnen rektifiziert wird, die beim Druck von 0,05 bis 4 kPa mit einer summarischen minimalen Wirksamkeit der Gruppe der Kolonnen von 10 theoretischen Geschossen arbeiten, wobei daraus durch die Rektifikation Leichtanteile und Schwerrückstände getrennt werden, wodurch davon ε-Caprolaktam isoliert wird.

2. Verfahren nach dem Anspruch 1, **dadurch gekennzeichnet, dass** die konzentrierte Mischung nach der Beseitigung einer festen Phase beim Druck von 0,3 bis 1 kPa destilliert wird.

3. Verfahren nach dem Anspruch 1, **dadurch gekennzeichnet, dass** eine Konzentration vom ε-Caprolaktam im Destillat durch einen Zusatz eines Lösungsmittels auf 40 bis 60 % Gew. aufbereitet wird.

4. Verfahren nach dem Anspruch 1, **dadurch gekennzeichnet, dass** ein Raffinat in einer Serie von Rektifikationskolonnen rektifiziert wird, die beim Druck von 0,1 bis 2 kPa arbeiten.

5. Verfahren nach dem Anspruch 1, **dadurch gekennzeichnet, dass** mindestens 5 % der Leichtanteile und Schwerrückstände, die durch eine Rektifikation vom Raffinat getrennt werden, in die obere Laktamphase zur weiteren Isolation vom ε-Caprolaktam zurück eingeführt werden.

6. Verfahren nach dem Anspruch 1, **dadurch gekennzeichnet, dass** ein Destillat bei einer Destillation partial kondensiert wird.

7. Verfahren nach dem Anspruch 1, **dadurch gekennzeichnet, dass** eine Hydrierraffination in der Anwesenheit eines heterogenen Trägerkatalysators stattfindet, der einen Träger auf Basis Al₂O₃ oder SiO₂ aufweist, auf dem mindestens ein Edelmetall der Gruppe Pd, Pt, Ru oberflächlich aufgetragen wird, wobei die Gesamtmenge eines Edelmetalls/der Edelmetalle 0,1 bis 5 % Gew. von diesem Katalysator darstellt.

8. Verfahren nach dem Anspruch 7, **dadurch gekennzeichnet, dass** die Gesamtmenge eines Edelmetalls/der Edelmetalle 1 bis 3 % Gew. vom Katalysator darstellt.

9. Verfahren nach dem Anspruch 1, **dadurch gekennzeichnet, dass** eine Hydrierraffination im Reaktor mit einer benetzten Katalysatorschicht stattfindet.

10. Verfahren nach dem Anspruch 7 oder 8, **dadurch gekennzeichnet, dass** ein Träger des Katalysators durch die Partikel mit einer Größe von 1 bis 5 mm gebildet wird.

## Revendications

1. Procédé de séparation de l'ε-caprolactame de la phase lactame supérieure obtenue par séparation de la phase sulfate après la neutralisation du mélange réactionnel obtenu par réarrangement de Beckmann du cyclohexyloxime avec de l'ammoniac, **caractérisé en ce que** cette phase lactame supérieure est d'abord distillée sous pression réduite ou atmosphérique, ce qui fait diminuer la contenance d'eau dans la phase à 5-10 % mas., puis la phase solide formée lors de la distillation est éliminée mécaniquement et le mélange concentré ainsi obtenu est distillé avec une pression de 0,1 à 4 kPa en éliminant les éléments non-volatiles, puis dans le distillat obtenu ainsi en ajoutant au moins un solvant du groupe eau, méthanol, éthanol, propanol, alcool isopropylique ou un mélange d'au moins deux d'entre eux, est ajustée la concentration en ε-caprolactame de 30 à 70 % mas., et le mélange obtenu ainsi est affiné par hydrogénation avec température de 100 à 150 °C et pression d'hydrogène de 1 à 10 MPa en présence d'un catalyseur hétérogène de support qui contient au moins un métal rare du groupe Pd, Pt, Ru, réduisant ainsi les liaisons multiples des impuretés présentes qui absorbent le rayonnement avec longueur d'onde de 290 nm, puis la solution est rectifiée dans une série d'au moins deux colonnes de rectification qui fonctionnent avec une pression de 0,05 à 4 kPa avec un rendement global minimal de l'ensemble de 10 colonnes à plateaux idéaux, séparant ainsi par rectification les fractions légères et les résidus lourds, isolant l'ε-caprolactame.

2. Procédé selon la revendication 1, **caractérisé en ce que** le mélange concentré est, après l'élimination de la phase solide, distillé sous pression de 0,3 à 1 kPa.

3. Procédé selon la revendication 1, **caractérisé en ce que** la concentration de l'ε-caprolactame dans le distillat est ajustée par l'ajout de solvant à la valeur de 40 à 60 % mas.

4. Procédé selon la revendication 1, **caractérisé en ce que** la solution est rectifiée dans une série de colonnes de rectification qui fonctionnent avec pression de 0,1 à 2 kPa.

5. Procédé selon la revendication 1, **caractérisé en ce que** au moins 5 % de fractions légères et de résidus lourds qui sont séparés par rectification de la solution sont réintroduits dans la phase lactame supérieure pour une nouvelle séparation de l'ε-caprolactame.

6. Procédé selon la revendication 1, **caractérisé en ce que** le distillat est, lors de la distillation, condensé partiellement.

7. Procédé selon la revendication 1, **caractérisé en ce que** l'affinage par hydrogénation s'effectue en présence d'un catalyseur hétérogène qui comprend un support à base de Al₂O₃ ou SiO₂, sur lequel au moins un métal rare du groupe Pd, Pt, Ru est revêtu en surface, tandis que la quantité totale du métal/métaux rare/s représente 0,1 à 5% de la masse de ce catalyseur.

8. Procédé selon la revendication 7, **caractérisé en ce que** la quantité du métal/métaux rare/s représente de 1 à 3 % de la masse du catalyseur.

9. Procédé selon la revendication 1, **caractérisé en ce que** l'affinage par hydrogénation s'effectue dans un réacteur avec catalyseur à biofiltre.

10. Procédé selon la revendication 7 ou 8, **caractérisé en ce que** le support du catalyseur est constitué de particules dont la taille varie de 1 à 5 mm.
